# EUROPEAN PATENT SPECIFICATION

(11) **EP 2 054 719 B1**
(45) Date of publication and mention of the grant of the patent: **23.07.2014**
(21) Application number: 07841195.6
(22) Date of filing: 22.08.2007
(51) Int. Cl.: G06F 19/22, C12Q 1/68

(54) **DESIGN AND SELECTION OF GENETIC TARGETS FOR SEQUENCE RESOLVED ORGANISM DETECTION AND IDENTIFICATION**
KONZEPTION UND AUSWAHL GENETISCHER TARGETS ZUR ERKENNUNG UND IDENTIFIKATION VON ORGANISMEN MIT AUFGELÖSTEN SEQUENZEN
CONCEPTION ET SÉLECTION DE CIBLES GÉNÉTIQUES POUR UNE DÉTECTION ET UNE IDENTIFICATION D'ORGANISME PAR RÉSOLUTION EN SÉQUENCE

(30) Priority: 22.08.2006 US 823101 P; 25.08.2006 US 823510 P; 14.11.2006 US 559513
(43) Date of publication of application: 06.05.2009
(73) Proprietor: The Government of the United States of America as represented by the Secretary of the Navy, Washington, DC 20375 (US)
(72) Inventor: MALANOSKI, Anthony, P., Washington, D.C. 20002 (US); WANG, Zheng, Burke, VA 22015 (US); LIN, Baochuan, Bethesda, MD 20814 (US); STENGER, David, A., Herndon, VA 20171 (US); SCHNUR, Joel, M., Burke, VA 22015 (US)
(74) Representative: Schumacher, Horst
(86) International application number: PCT/US2007/076499
(87) International publication number: WO 2008/024827

(56) References cited:
- US-A1- 2003 228 599
- US-A1- 2005 227 222
- Y. ZHAN ET AL: "Model-P: a basecalling method for resequencing microarrays of diploid samples", BIOINFORMATICS, vol. 21, no. Suppl 2, 1 September 2005 (2005-09-01), pages ii182-ii189, XP055053039, ISSN: 1367-4803, DOI: 10.1093/bioinformatics/bti1129
- CUTLER DAVID J ET AL: "High-throughput variation detection and genotyping using microarrays", GENOME RESEARCH, COLD SPRING HARBOR LABORATORY PRESS, WOODBURY, NY, US, vol. 11, no. 11, 1 November 2001 (2001-11-01), pages 1913-1925, XP002311211, ISSN: 1088-9051
- C. WU: "Sequence dependence of cross-hybridization on short oligo microarrays", NUCLEIC ACIDS RESEARCH, vol. 33, no. 9, 16 May 2005 (2005-05-16), pages e84-e84, XP055052885, ISSN: 0305-1048, DOI: 10.1093/nar/gni082
- O. V. MATVEEVA: "Thermodynamic calculations and statistical correlations for oligo-probes design", NUCLEIC ACIDS RESEARCH, vol. 31, no. 14, 15 July 2003 (2003-07-15) , pages 4211-4217, XP055052884, ISSN: 0305-1048, DOI: 10.1093/nar/gkg476
- A. P. MALANOSKI ET AL: "A model of base-call resolution on broad-spectrum pathogen detection resequencing DNA microarrays", NUCLEIC ACIDS RESEARCH, vol. 36, no. 10, 1 January 2008 (2008-01-01), pages 3194-3201, XP055052865, ISSN: 0305-1048, DOI: 10.1093/nar/gkm1156

## Description

This application claims the benefit of US Provisional Patent Application Nos. 60/823,101, filed on 08/22/2006 and 60/823,510, filed on 08/25/2006. This application is a continuation-in-part application of US Patent Application Nos. 11/177,646 filed on 07/02/2005, 11/177,647 filed on 07/02/2005, 11/268,373 filed on 11/07/2005, 11/422,425 filed on 06/06/2006, 11/422,431 filed on 06/06/2006, and 11/559,513 filed on 11/14/2006. These applications claim priority to US Provisional Patent Application Nos. 60/590,931 filed on 07/02/2004, 60/609,918 filed on 09/15/2004, 60/626,500 filed on 11/05/2004, 60/631,437 filed on 11/29/2004, 60/631,460 filed on 11/29/2004, 60/735,824 filed on 11/14/2005, 60/735,876 filed on 11/14/2005, 60/743,639 filed on 03/22/2006, and 60/691,768 filed on 06/16/2005.

### TECHNICAL FIELD

The invention is generally related to resequencing microarray design.

### BACKGROUND ART

As the prevalence of DNA based detection methods increases, it becomes more important to have *in silico* methods to design, test, and improve the analysis of assays. In particular, highly multiplexed pathogen detection is a growing requirement and is potentially more efficient than multiple separate tests in costs, required sample volumes, reagents, and assay time. However, the initial development, design, and validation can become logarithmically complex, costly, and time consuming. Accurate simulation models using newly available genetic sequence information for microorganisms can potentially minimize costs and time of developing these highly multiplexed assays.

The design criteria for all nucleic acid-based assays have similar global constraints. After the target organisms are chosen, methods must be employed to choose probes that will very specifically recognize only the target organism species and yet account for all of the genetic variations (i.e. strains or subtypes) within that species. *In silico* design methods have been developed for PCR and spotted oligonucleotide microarrays (Cleland et al. (2004) Development of rationally designed nucleic acid signatures for microbial pathogens. Expert Rev Mol Diagn, 4, 303-315; Gardner et al. (2005) Draft versus finished sequence data for DNA and protein diagnostic signature development. Nucleic Acids Res, 33, 5838-5850; Rychlik et al. (1989) A computer program for choosing optimal oligonucleotides for filter hybridization, sequencing and in vitro amplification of DNA. Nucleic Acids Res, 17, 8543-8551; Fitch et al. (2002) Rapid development of nucleic acid diagnostics. Proceedings of the IEEE, 90, 1708-1721) assays and oligonucleotide microarrays (Herold et al. (2003) Oligo Design: a computer program for development of probes for oligonucleotide microarrays. Biotechniques, 35, 1216-1221; Mehlmann et al. (2006) Robust sequence selection method used to develop the FluChip diagnostic microarray for influenza virus. J Clin Microbiol, 44, 2857-2862), with the models for each having similar requirements. Because the potential pool of probes, targets, and interference fragments is so large, models that result in maximal target specificity with minimal computation are preferred. In typical PCR primer or oligonucleotide microarray design algorithms, the number of base matches is counted between a probe and a target or background organism sequence. If a threshold number of matches is exceeded then hybridization is assumed (Herold et al. (2003) Oligo Design: a computer program for development of probes for oligonucleotide microarrays. Biotechniques, 35, 1216-1221; Mehlmann et al. (2006) Robust sequence selection method used to develop the FluChip diagnostic microarray for influenza virus. J Clin Microbiol, 44, 2857-2862.). This level of modeling is incomplete because the ultimate detection of the probe-target hybridization depends on a single signal intensity (usually fluorescence), which may not correlate with that predicted. This results in uncertainty about how effective the selected probes will be until experimental work is performed to validate the selections and establish intensity cutoffs for hybridization events.

More detailed thermodynamic modeling and calculations have been used to better understand match-mismatch and single match microarrays and allow predictions of intensity (Matveeva et al. (2003) Thermodynamic calculations and statistical correlations for oligo-probes design. Nucleic Acids Res, 31, 4211-4217; Held et al. (2003) Modeling of DNA microarray data by using physical properties of hybridization. Proc Natl Acad Sci USA, 100, 7575-7580; Naef et al. (2003) Solving the riddle of the bright mismatches: Labeling and effective binding in oligonucleotide arrays. Physical Review E, 68, 011906; Zhang et al. (2003) A model of molecular interactions on short oligonucleotide microarrays. Nat Biotechrtol, 21, 818-821; Wu et al. (2005) Sequence dependence of cross-hybridization on short oligo microarrays. Nuclei Acids Res, 33, e84). The modeling approaches account for several important issues such as probe attachment to the surface, and the effect of dimer formation of the fragments or loop formation depending on the base content of the fragments. Accounting for these issues when only one or two probes might hybridize with a target is relatively straightforward. However this increased detail in the model comes at a price in that the computational requirements also increase.

In contrast to simple oligonucleotide microarrays, recent work using resequencing microarrays demonstrated that they are a viable alternative to test for multiple pathogens, including co-infections, and perform detailed discrimination of closely related pathogens and/or track pathogen mutation (Wang et al. (2006) Identifying Influenza Viruses with Resequencing Microarrays. Emerg Infect Dis, 12, 638-646; Lin et al. (2006) Broad-spectrum respiratory tract pathogen identification using resequencing DNA microarrays. Genome Res, 16, 527-535). Because sets of 4 (or 8 if anti-sense is also included) short probes, where each set represents a portion of desired sequence and all the variations of the center nucleotide position, the absolute intensity of signal from a single probe becomes less important than the differential binding/intensity across the complete probe set. This information, confirmed in both the sense and antisense directions, is used only to determine that a particular base is present with high confidence. This use of overlapping probe sets is required to *directly* determine a target organism's nucleotide sequence, not *inferentially* based on single fluorescent signal intensities of presumably specific probes (Malanoski et al. (2006) Automated identification of multiple microorganisms from resequencing DNA microarrays. Nucleic Acids Res, 34, 5300-5311).

A resequencing microarray's effectiveness for broad spectrum detection of various levels of organism discrimination may be dependent on the process used to select the reference or target sequences placed on the microarray. Tradeoffs in amount of space dedicated to an organism versus the level of discrimination possible must be balanced for every organism considered. In addition, when specific or semi-specific primers are used for organism enrichment the selection of these primers can affect the possible reference sequence selections.

The overall design process can be characterized as a series of steps. First, selection of organisms and desired level of discrimination for each organism and whether specific nucleic acid markers must be tested for. Second, determination from known sequence data of sequence regions to choose reference sequences from. Third, selection of reference sequences and check for possible conflicts. Fourth, primer selection. Fifth, refinements of sequence selections. The order of several of these steps can be interchanged and refinements consist of repeating several of these steps after making changes. The first step is always the selection of organisms and the desired discrimination levels of each organism which represent constraints on the design. The size of the microarray to be used specifies the other constraint placed on the design problem. It may be that no solution is possible without altering one or more of the constraints. But all subsequent steps are aimed at meeting these requirements.

### DISCLOSURE OF THE INVENTION

The invention comprises a computer-implemented method comprising: providing a list of target sequences associated with one or more organisms in a list of organisms; providing a list of candidate prototype sequences suspected of hybridizing to one or more of the target sequences; generating a collection of probes corresponding to each candidate prototype sequence, each collection of probes comprising a set of probes for every subsequence having a predetermined, fixed subsequence length of the corresponding candidate prototype sequence, the set consisting of the corresponding subsequence and every variation of the corresponding subsequence formed by varying a center nucleotide of the corresponding subsequence; generating a set of fragments corresponding to each target sequence, each set of fragments comprising every fragment having a predetermined, fixed fragment length of the corresponding target sequence; calculating the binding free energy of each fragment with a perfect complimentary sequence of the fragment, and if any binding free energy is above a predetermined, fixed threshold, the fragment is extended one nucleotide at a time until the binding free energy is below the threshold or the fragment is the same length as the probe, generating a set of extended fragments; and determining which extended fragments are perfect matches to any of the probes; and assembling a base call sequence corresponding to each candidate prototype sequence comprising: a base call corresponding to the center nucleotide of each probe of the corresponding prototype sequence that is a perfect match to any extended fragment, but for which the other members of the set of probes containing the perfect match probe are not perfect matches to any extended fragment; and a non-base call in all other circumstances.

### BRIEF DESCRIPTION OF THE DRAWINGS

A more complete appreciation of the invention will be readily obtained by reference to the following Description of the Example Embodiments and the accompanying drawings.

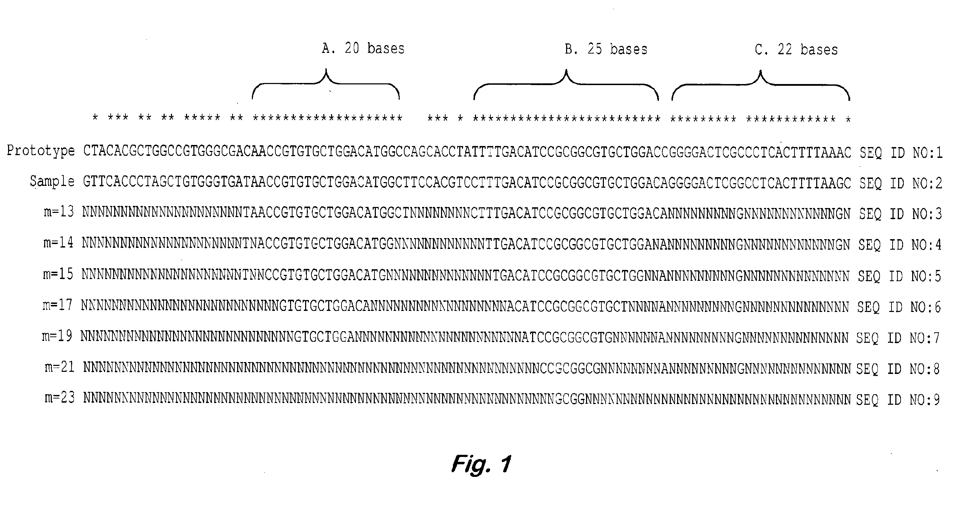

Fig. 1 shows example results of the model using different values of m from 23 to 13. A prototype sequence (used to make probe sets) and a sample sequence are shown with an asterisk above the bases that match in both sequences. Also shown are the reassembled model base call results for each probe set for different values of *m*. Region A has 20 contiguous bases so for *m* greater than 20 no probe sets in this region have matches. The longer region B has probe sets that make base calls at *m* = 23. For each region, an increase of one or two in *m* results in one or two base calls at each edge to cease making base calls. These base calls depend on fragments that have more matches on one half of the probe than the other. Region C has two contiguous regions of 9 and 12 bases with a SNP in between. One probe of the SNP set has 22 bases that match in the sample but no other probe in any probe set in the region has more than 12 matching and so all are N calls at all values on N.
Fig. 2 shows the frequency of resolved base calls from primers as a function of position within the primer. ●- All, GC content: ▲- less than 50%, ▼-more than 50%.
Fig. 3 shows the frequency of resolved base calls from primers as a function of position within the primer. ΔG (open symbols indicate bin with fewer than 12000 data points): *>-13, -13>■□>-16, -16>◆◊>-19, -19>▲△>-22, -22>▼∇>-25, -25>●○

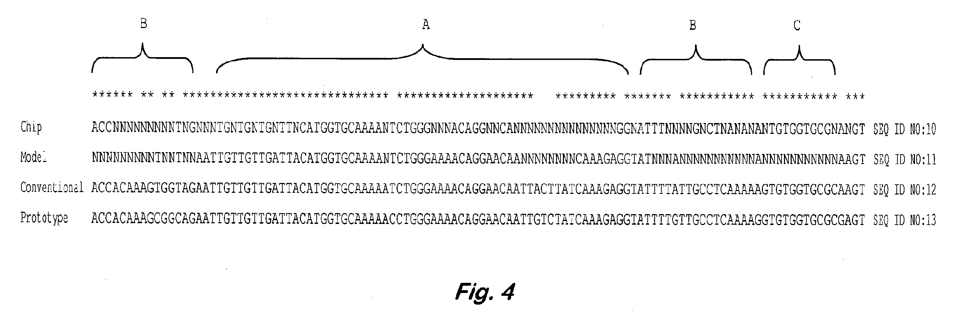

Fig. 4 shows the prototype sequence of FIuBHA and results for an influenza B Victoria lineage sample from conventional sequencing, from RPMv.1 microarray, and from model prediction. Region A represents a section sequence where SNPs are very far apart or close together and the model and microarray data agree well. Region B sequences have SNPs with an intermediate frequency and the agreement between model and experiment decreases. This behavior observed as the percent difference between sample and prototype sequence rises above 4%. Region C is similar although the number of observed base calls observed is much higher and these cases were only observed at 10%.
Fig. 5 shows a hypothetical nominal target, list of targets, and list of prototype sequences.
Fig. 6 shows a hypothetical collection of probes.
Fig. 7 shows hypothetical lists of fragments and extended fragments.
Fig. 8 shows the perfect matches between the probes and the extended fragments.
Fig. 9 shows hypothetical base call sequences.
Fig. 10 shows the matching organisms for each candidate prototype and formation of the list of final targets.

### MODES FOR CARRYING OUT THE INVENTION

In the following description, for purposes of explanation and not limitation, specific details are set forth in order to provide a thorough understanding of the present invention. However, it will be apparent to one skilled in the art that the present invention may be practiced in other embodiments that depart from these specific details. In other instances, detailed descriptions of well-known methods and devices are omitted so as to not obscure the description of the present invention with unnecessary detail.

The prevalence of DNA based detection methods, particularly for multiple pathogen detection, is evident from the volume of recently published literature. Thus, it becomes important to have *in silico* methods to assist in the design, initial test, and improvement of these methods as their development becomes more complex, costly, and time consuming. Recent work using resequencing microarrays demonstrates that they are a viable alternative to test for multiple pathogens, including co-infections, as well as performing detailed discrimination of closely related pathogens and/or track pathogens' genetic variations. However, the qualities of resequencing arrays require that different criteria are needed for modeling their performance at the individual probe level. In addition, optimizing the design of these assays with potentially hundreds of prototype targets exceeds what is possible by current methods. To address these issues, a computationally efficient model for predicting base calling for resequencing microarrays was successfully developed that begin with a simple assumption to predict hybridization and then only added complexity as needed. A large set of data for organism and short oligonucleotide hybridization and base calling with Affymetrix CustomSeq microarrays allowed testing and validation of the model.

Disclosed is a model applicable to resequencing microarrays that predicts the base calls that will occur for a sample sequence on a specified prototype sequence of the microarray. A "prototype" sequence is the designation for the genomic sequence used to generate the probe sets placed on the resequencing array allowing at least partial hybridization of a selected range of pathogen target sequences. Although rules similar to those used in designing for other arrays are the starting point to allow rapid calculations, more detailed thermodynamic information is incorporated. The model development is facilitated by testing against a large set of data for organisms and short oligonucleotide hybridizations and base calling on Affymetrix resequencing microarrays. The model is successful at predicting base calls from hybridization of a large variety of target organism sequences. It can further be used to predict how well prototype sequences represented on the microarray will perform against a diverse set of pathogen targets. This will assist in simplifying the design of resequencing microarrays and reduce the time and costs required for their development for specific applications.

*Model Concept -* Experimentally, a probe set will only indicate that a specific base is present if a fragment binds better to one probe of the set. To model this behavior, the central assumption made is that when a probe and a sample sequence have *m* contiguous bases that complement, an observable hybridization signal occurs. This is the roughest approximation to represent the difference in binding strengths of different sequences to a probe and represents the simplest model. The remainder of the modeling consists of generating probes from the prototype sequence and potential binding fragments from the sample, and then comparing the sets with each other using the central assumption.

The first step is to generate the probe sets and sample fragments. A sequence selected to be the prototype sequence is divided into overlapping sets of 4 probes, where the probes of a set are each, for example, 25 bases long and differ at the central base (i.e. for a sequence of L bases, L-24 probe sets are produced). This represents what may actually be located on a microarray. For a sample sequence, all unique fragments that are *m* bases long are generated (i.e. for a sequence of *K* bases, at most *K*-*m*+1 unique fragments can be produced). Fragments in an experiment may be longer than this (average of 100 bases). The model only requires that the minimum requirement of *m* bases be present in a fragment.

Now that the microarray probes and sample fragments have been generated, each probe of every probe set is tested against all the fragments from the sample sequence to determine if a perfect complement match occurs. Probes having a match are noted. The ability of a probe set to produce a base call is evaluated by considering the results of its probes. If only one probe of the set has a match in the sample sequence, that is the base call assigned for the probe set and the next probe set is examined. N, representing an ambiguous base identity, is assigned when none of the sample fragments are a match to any member of the probe set. In the case that more than one probes of a set has a match, longer fragments are generated from the sample sequence and then compared. The neighboring bases of each fragment in the 5'-3' direction from the sample sequence are added to one at a time until a mismatch occurs with the appropriate probe. If one of these fragments is now longer than the others, then that base is assigned, otherwise N is assigned.

After all probe sets are tested, the base calls (A, C, T, G, or N) from each probe set are reassembled into a sequence. Fig. 1 shows example results of the model using different values of *m* from 23 to 13 (lengths less than 13 were not used as they can bind nonspecifically, though it is possible to use them) and points out some base calls made under various conditions. Although experimental results clearly indicate it is not necessary for a fragment to complement all 25 or even 21 bases of a probe to produce a specific base call. Without further experimental input, it is difficult to determine what length for m is most appropriate.

*Short Oligomers -* A large amount of data on the hybridization of short oligonucleotides was available from Respiratory Pathogen Microarray v.1 (RPMv.1) (Lin et al. (2006) Broad-spectrum respiratory tract pathogen identification using resequencing DNA microarrays. Genome Res, 16, 527-535) experiments using a multiplex of specific primers for sample amplification. Since unused primers were not removed from the sample before hybridization and most of these primers were within the prototype sequences, it is possible to study the binding of a large number of short oligomers 16 to 27 bases in length to resequencing microarrays. The data sets are for two multiplex mixtures, one contains 117 primers (777 experiments) and the other (906 experiments) consists of 66 primers that are a subset of the 117-primer mixture. There are multiple probe sets available from the prototype sequence that will hybridize with the same primer but have a different number of bases that exactly match available for hybridizing (from 13 bases to the length of the primer or the length of the probe, 25 bases). For example, the base at either end of the primer oligomer has a probe set that may determine the identity of the base but only based on hybridization of 13 bases. The primers of any prototype sequence that showed better than 50 percent hybridization for its entire sequence were not included in the analysis as they represent hybridization of unused primer and primer incorporated into amplicons of the target. From the collection of primer oligomers available there were ~3 x 10⁵ data points for each length from 13 to 21, ~2 x 10⁵ for 22, ~1.5 x 10⁵ for 23 and ~7.5 x 10⁴ for each length of 24 and 25. Base calling was performed by GDAS program settings used in previous work (Lin et al. (2006) Broad-spectrum respiratory tract pathogen identification using resequencing DNA microarrays. Genome Res, 16, 527-535).

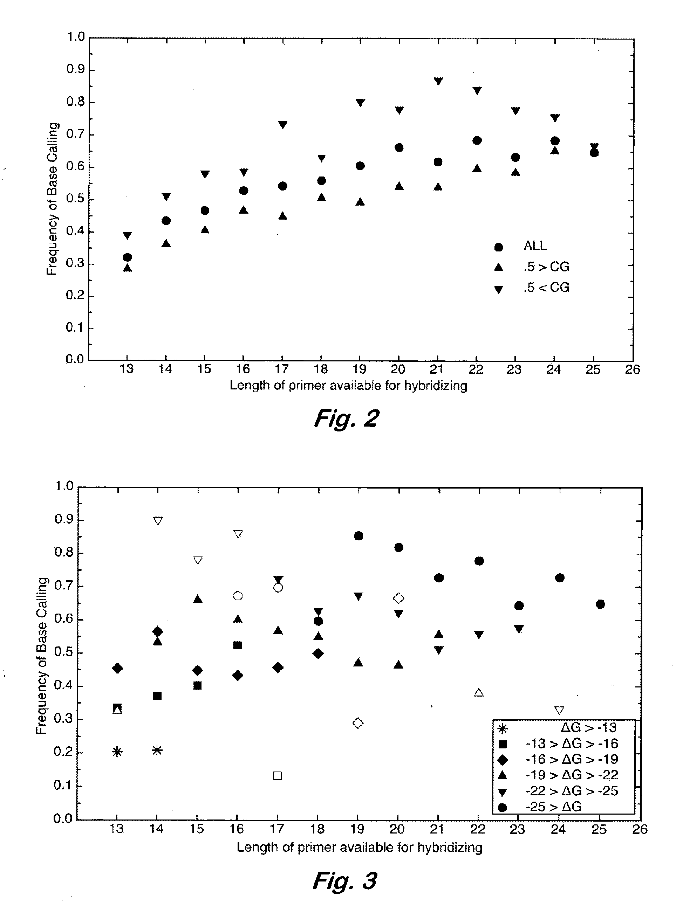

Fig. 2 shows the frequency of an unambiguous base call versus the amount of primer that can hybridize to a probe for all primers and two groups of primers based on their GC content. The first position has a frequency of 33% which indicates that 1 time in 3 a DNA fragment that only matches 13 of the 25 bases in a probe is able to bind specifically and strongly enough to generate a unique base call. As the length of bases available to hybridize increases, an increasing frequency of base calling is observed and reaches 50% or more by a length of 16. To further understand the binding frequency, the results of the multiplex primers hybridization were divided into two groups based on their GC content. The averages for primers are shown grouped with GC contents less than 50% and greater than or equal to 50%. This division places roughly twice the number of samples in the lower bracket than are in the upper bracket for lengths up to 22. The difference in frequency of base calling is largest going from 13 to 14. The rates and trend from 23 to 25 for GC content greater than 50% has greater uncertainty, as there are significantly fewer probe samples in these brackets.

To understand the influence of primer composition better, Fig. 3 shows the primers of each length in separate groups based on the ΔG calculated by the nn model (SantaLucia (1998) A unified view of polymer, dumbbell, and oligonucleotide DNA nearest-neighbor thermodynamics. Proc. Natal. Acad. Sci. U S A, 95, 1460-1465; SantaLucia et al. (2004) The thermodynamics of DNA structural motifs. Annu. Rev. Biophys. Biomol. Struct., 33, 415-440). Some of these bins have very few samples and those results exhibit greater uncertainty. Nevertheless, a trend can be observed that overall as the ΔG decreases, the frequency increases irrespective of the length. The interesting point is using one perfect match and three mismatch probes a high base call frequency is possible for oligomer lengths significantly shorter than the length of the probes (25 bases). The only probes that clearly have a low frequency of making base call on the array have lengths of 13 and 14 and ΔG greater than -13 kcal/mol. Primers with ΔG lower than -16 kcal/mol on average have 50 percent or greater chance to hybridize and produce a base call.

*Revised Model Concept -* The experimental evidence from the trend in the binding frequencies indicates that lengths longer than 16 are likely to frequently generate a resolved base call without considering any other factors. For shorter lengths, the ΔG of the probe is important in determining if there will be a significant chance of resolving base call. The model was modified to determine the ΔG of the fragments generated from the sample with *m* =13. If the fragment's free energy difference is below the cutoff, -14.5 kcal/mol, it is accepted. In the case it is above the cutoff, the length of the fragment is increased until its energy is below the cutoff or it reaches the length of a probe, 25. The resulting list of fragments is then compared against every probe set as already mentioned.

*Amplification, hybridization, and sequence determination* - The details of the Respiratory Pathogen Microarray v.1 (RPM v.1) design and the experimental methods have been discussed in previous work (Wang et al. (2006) Identifying Influenza Viruses with Resequencing Microarrays. Emerg Infect Dis, 12, 638-646; Lin et al. (2006) Broad-spectrum respiratory tract pathogen identification using resequencing DNA microarrays. Genome Res, 16, 527-535; Davignon et al. (2005) Use of resequencing oligonucleotide microarrays for identification of Streptococcus pyogenes and associated antibiotic resistance determinants. J Clin Microbiol, 43, 5690-5695; Lin et al. (2007) Using a Resequencing Microarray as a Multiple Respiratory Pathogen Detection Assay. J Clin Microbiol., 45(2), 443-452). Partial sequences from the genes containing diagnostic regions were tiled for the detection of these pathogens. The experimental microarray data used for the initial primer analysis were obtained from clinical samples using multiplexed RT-PCR amplification schemes. The results for test of primer results and the California lineage samples used a different multiplex protocol (Lin et al. (2007) J Clin Microbiol., 45(2), 443-452). The remaining influenza samples used a random protocol (Wang et al. (2006) Emerg Infect Dis, 12, 638-646). GCOS™ software v1.3 (Affymetrix Inc., Santa Clara, CA) was used to determine the intensities of the probes and the base calls were made using GDAS v3.0.2.8 software (Affymetrix Inc., Santa Clara, CA).

*Case 1: Predicting Primer Interference -* The first test use of the model algorithm was to understand base calling that was occurring in 42 microarray experiments with a blank sample (no nucleic acids added) using a new primer set that tried to minimize the primer interaction with the prototype sequences. Since the primers were still present, they were treated as collection of sample sequences and tested using the model against every prototype sequence on the chip. The model accurately predicted the base calling occurring in the experiments from primers that were still located on the prototype sequences. Additional binding to locations in the center of prototype sequences was also seen and agreed with the experimental results. Primers designed for prototype sequences of closely related organisms caused these base calls. For example, the adenovirus 4 *E1A* gene prototype sequence has 19 of 20 predicted bases being called 97% of the time, which is located 393 bases from the beginning of the sequence. One base, which is a single nucleotide polymorphism (SNP) at the edge of the region, was predicted to call was but was observed only called 12% of the time in the experiments. This region when compared to other prototype sequences is a match for primer region selected for the adenovirus 7 *E1A* prototype region. Similar agreement was seen for the other 47 regions predicted by the model.

*Case 2: Model Predictions for Long Sequellces -* After successful demonstration of the accuracy of the model for shorter fragments, the predictions for entire prototype sequences were examined. Results using conventional sequencing samples in the model compared to experimental microarray results for four data sets; influenza A/H3N2 Fujian-like lineage, influenza A/H3N2 California-like lineage, influenza B Yamagata/16/88 lineage, and influenza B Victoria/2/87 are reported in Table 1. The results report averages for samples that have a great deal of similarity such as for the influenza A/H3N2 Fujian-like samples, the average base call rate for the experiments was 85% while the model predictions averaged 97%. The average number of SNPs was 9.8 (1%) between the prototype and the conventional sequences. While the model predicted 9.2 SNPs would be resolved, only 6.3 SNPs were observed in the experiments. The model predicts 8.8 N calls that the experiment has a specific base call, and the microarray has 94.9 N calls that the model predicts should be a specific base call. So on average 14.3 N calls match between model and microarray results.

**Table 1 - Summary of average model and experimental microarray results for influenza hemagglutinin gene that could be placed in separate groups based on lineage.**

| Sample Set | Tile | Resolved Base calls | | Number SNPs with respect to Prototype | | | Number N calls | | |
|---|---|---|---|---|---|---|---|---|---|
| Influenza | | Array | Model | Conv | Model | Array | Model only | Array only | Model and Array |
| A Fujian-like lineage (12) | 770 | 85.4±3.6 | 96.7±0.012 | 9.8 | 9.2 | 9.2(0)* | 8.8 | 94.9 | 14.6 |
| A California-like lineage (12) | 770 | 92.2±7.8 | 95.3±0.013 | 11.9 | 11.6 | 10.7(1)* | 15.3 | 38.7 | 21.5 |
| B Yamagata lineage (8) | 660 | 77.5±3.7 | 86.8±0.011 | 24.5 | 17.6 | 12.2(1)* | 26.4 | 87.2 | 61 |
| B Victoria Lineage (4) | 660 | 47.7±3.9 | 51.4±0.007 | 65.2 | 39.2 | 31.2(4)* | 70.2 | 94.2 | 251 |

| | | | | | | | | | |
|---|---|---|---|---|---|---|---|---|---|
| * numbers in parentheses are number of disagreements with respect to conventional results | | | | | | | | | |

Table 2 shows for a specific isolate from the Fujian-like lineage samples (identified as A/Nepal/1727/2004) the location of each of 6 SNPs resolved on the microarray and the number of additional bases that were called N in a 25 base long window centered on the SNPs. The total base call rates were 97.4% for the model and 88.4% for the microarray. Using this information to group the N calls, 46 N calls are closely related with SNPs and 29 N calls are spread uniformly across the microarray and mostly consisted of single N calls surrounded by resolved bases or a few events of two consecutive N calls or two N calls in a group of three bases. The sample has a total of 8 SNPs when comparing the conventional and prototype sequences and the two SNPs not identified on the microarray were both located near other SNPs that were identified. The model and microarray agree on 12 N calls located near 7 different SNPs but six more N calls predicted in the model near SNPs were resolved in the experiment and so represent discrepancies in the model.

**Table 2 - Location of SNP for influenza A strain compared to FluAHA3 prototype sequence.**

| Location | Target Base | Actual Base | N calls in local region(chip) | N calls in local region (model) |
|---|---|---|---|---|
| 299 | G | A | 10 | 1 |
| 313 | G | A | 8 | 1 |
| 352 | A | C | 10 | 8 |
| 393 | A | T | 2 | 3 |
| 483 | G | A | 5 | 0 |
| 593 | G | A | 8 | 3 |
| 596 | T | C | 8 | 3 |
| 698 | C | A | 3 | 4 |

The prototype sequence differed from the sample sequence by 1.5% for the influenza A/H3N2 California-like lineage samples and 3.7% for the influenza B Yamagata/16/88 lineage samples and 9.8% for the influenza B Victoria/2/87 lineage samples. These results differed from the first group of samples also in that there were disagreements between the conventional sequencing and the microarray base calls other than N calls. The influenza B samples that were run under the same protocol as the influenza A/H3N2 Fujian-like lineage had 1 (Yamagata lineage) and 4 (Victoria lineage) base call differences. These bases calls all occurred in regions at least 3 N calls from any region of many resolved base calls and the model predicted N base calls at these locations. The influenza A/H3N2 California-like samples used a different protocol and while the disagreements have many N calls near them, they do not consistently have at least 3 N calls separating them from regions of many resolved bases. This accuracy of 99.87% on the bases calls is a reasonable error rate to expect when determining the base calls from a single microarray experiment.

The model has a similar performance for the percentage of base calls predicted for samples that differ from the prototype sequence from 1% to 4% and appears to have a slightly better agreement when the difference increase to ~10%. However, overall base call percentage can be a misleading indicator of model performance. The N calls can be broken down into three groups; N calls predicted in model but not observed, N calls observed but not predicted, and N calls both predicted and observed. Examining the trends one can see that for the three sample sets subject to the same protocol as the amount of variation increased from 1% to 10%, the predicted N calls that matched observed N calls increased by the largest amount reflecting where the model is accurate. The N calls observed but not predicted remains roughly constant. The N calls made in the model but that are resolved base calls on the chip also increases. The improved agreement for the percentage of base calls seen at 10% is caused by the increase overall base call. Overall the other influenza A/H3N2 sample behaves in a similar manner to the other data sets and the differences in some details probably reflect differences in the protocol used. Even though the model is not as accurate when SNPs occur more frequently, the regions that have a lower frequency are correctly identified and these are the regions that are used in our current pathogen identification analysis. Fig. 4 shows a section from an influenza B sample that differs by 10%. Some features like the large stretches of N calls or resolved calls are present in all sample sets. The stretches of base calls from these regions are what are used most often in the analysis program, CIBSI v.2. The B regions of Fig. 4 represent scattered base calls in a region of predicted N calls and are found in the sample sets having 4% or more variation. The C region in Fig. 4 is similar to region B except in this case many more experimentally resolved base calls in the region are predicted as N. This type of behavior was only observed in the samples of 10% variation.

The model can be used to understand the behavior of an organism when using a representative sequence from a genomic sequence database rather than the conventional sequencing of the sample. An example is the influenza A/Puerto Rico/8/34 strain was used as a spike in test on the microarray and the experiments only had significant base call rates on the *neuraminidase* and *matrix* prototype sequences. This is consistent with the model simulation which correctly identified the regions in the two prototype sequences that would generate significant base calls and predicted that an insignificant number of base calls would occur in the *hemagglutinin* prototype sequence due to differences between the influenza A/Puerto Rico/8/34 strain and prototype sequence.

The examination of a large collection of resequencing microarray probe sets using well defined short oligomer probes has clearly demonstrated that short fragments with only 16 sequential complementary bases can produce accurate base discrimination a significant fraction of the time. This hybridization is independent of GC content or calculated ΔG, and segments as short as 13 bases will produce calls when the GC content or ΔG is favorable. The simple model for predicting hybridization patterns developed in this study has excellent agreement with observed experimental results when it was assumed that only 13 contiguous bases matching perfectly are required for specific binding. Better agreement was reached by also requiring that the predicted size of ΔG of a binding fragment meet a minimal size requirement. The implication for resequencing microarrays is that significant amounts of specific hybridization occurs, with resultant nucleotide base calling, for fragments that have less than a perfect 25 base match with the probes. The testing of the primers demonstrated the difficulties in eliminating all potential cross-hybridization of primers with prototype sequences in highly multiplexed systems. However, because probe-target hybridization on the microarray can be predicted, it is straightforward to account for cross-hybridization effects when analyzing the results and does not need to be physically eliminated. The model performs reasonably well, particularly for the application that drove its development and has provided insight into why this detection method works in complex mixtures. It should be applicable for predicting behavior of other microarrays that use complete match-mismatch probe sets with different criteria to select the probe sets, such as Affymetrix Mapping Arrays and Genotyping Arrays.

When considering the influenza B samples, it becomes apparent that some fragments that could potentially bind to probes might be missed when 13 contiguous complementary bases are required for hybridization. The evidence also suggests that fragments containing one mismatch with sufficiently strong binding energy can result in base calls. Unfortunately, the few samples of influenza B currently available make it impractical to try to establish what energy a fragment must have when it contains a mismatch. Another shortcoming of the model relates to its failure to predict N calls that are not closely associated with a SNP. Experimental microarray results provide only one microarray result per sample. Thus, it cannot be determined whether the scattered N calls appear reproducibly or randomly as many factors might influence this behavior. The formation of self-loop structures was eliminated as a dominant factor in the model, since incorporation of thus did not result in matching prediction and observed experimental patterns.

The current model can be used to predict whether sufficient base calls will occur for a pathogen of interest within a selected prototype sequence to be identified using the analysis program, CIBSI V2.0 (Malanoski et al. (2006) Automated identification of multiple microorganisms from resequencing DNA microarrays. Nuclei Acids Res., 34, 5300-5311). A simple rule of thumb can be made that sequences that differ by more than 80 percent from the probe sequence have few instances in which sufficient matching bases are contiguous to allow a significant amount of base calling and will never generate organism identification by our methods. This is a useful quick estimate of the upper bound on the maximum number of reference strains a probe sequence can detect. The developed model can be applied to the sequences that fall within this range to more accurately predict which organisms can be detected and the performance of a prototype sequence.

The results of the modeling can be used for selection of the prototypes for inclusion on a microarray. The overall design process can be implemented in the next microarray designs for biothreat agents and a regional (e.g. Africa) organisms specific microarray. The identification of the regions from organisms may or may not be solely a literature search. This will remain an important tool for larger genome targets but may be unnecessary for viral organisms with smaller genomes. The methodology for organism detection that will be applicable for any design can be characterized as a series of steps. First, the list of sequences is to include target sequences and any sequences from near genetic neighbors so that the effect of their hybridization to the reference sequences can be checked. A gross predictor of hybridization can be obtained from the percentage of bases that match an alignment procedure (BLAST). By using cutoff criteria below the percentage that commonly gives the smallest usable hybridization program, it is possible from BLAST queries to construct a list of sequences that may potentially hybridize in different regions. This list of sequences is to include target sequences and any sequences from near genetic neighbors so that the effect of their hybridization to the reference sequences can be checked. Second, coupling sequence selection with taxonomic information each region can be evaluated for whether it can give the desired level of discrimination and whether it limits its detection to desired targets only or not. This will provides an immediate upper limit on the possible number of organisms a reference sequence may usefully detect. Third, after the best candidate regions are determined using the above methods. Fourth, a list of the number of strains each strain can detect is made and used as the criteria for selecting reference strains. Fifth, the strain that detects the most other strains is removed from the list and used as the first reference strain. All strains that it is capable of detecting are also removed from the list. Of the remaining strains, the one that detects the most other strains is selected as the next reference strain. In the general formulation rather than limiting comparison to sequences only with the target, each of the sequences that need to be detected is tested as a potential reference sequence. The other organism sequences it can potentially identify will be obtained from a query using BLAST to determine which subset of the sequences has a chance of hybridizing. This subset is simulated with the more detailed model to predict hybridization. The resulting hybridization is evaluated using the detection algorithm developed to classify hybridization on real chips rather then the simpler criteria used before. For each potential reference sequence, a refined upper bound on the number of target and non-target sequences each can detect can now be established. Selection of reference sequences used will then proceed in a manner to use the minimum space to provide the required level of discrimination. Primer selection is then performed after the sequences have been selected.

The method may have the following features. The method does not rely on open literature solely to determine the reference sequences selection as they may be outdated from the addition of new organism sequence since the publication. The design scheme provides an independent check on the validity of the reference sequences selected before fabrication is carried out. The may be improvement over selected reference sequences which were possible only between microarrays designs based upon the performance of previous chip design. The method may determine a smaller set of reference sequences that can provide the level of discrimination specified without prior validation. The method may allow for an automation process for target gene selections and shorten the turn around time for chip design.

Having described the invention, the following examples are given to illustrate specific applications of the invention. These specific examples are not intended to limit the scope of the invention described in this application.

### Example 1

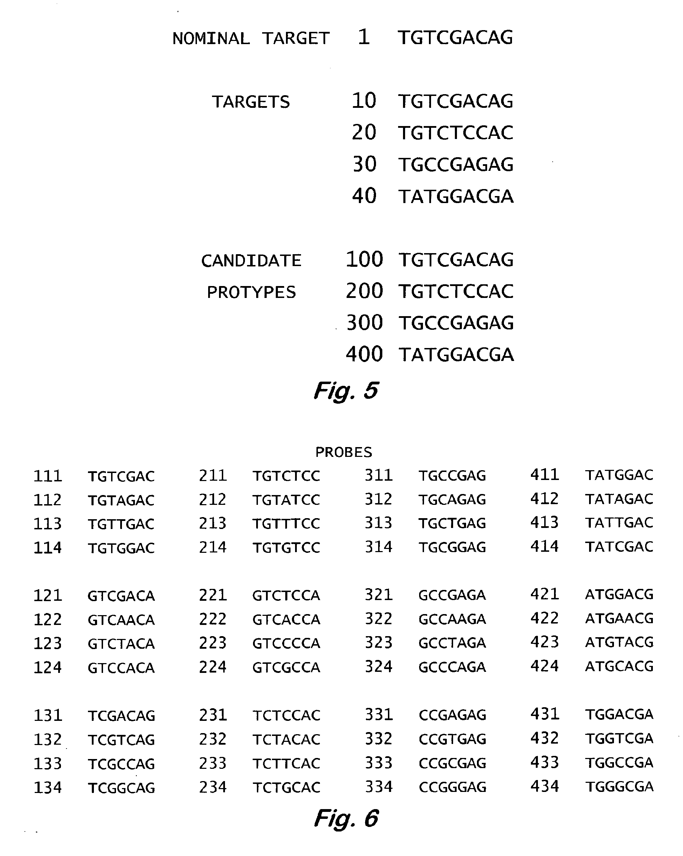

**Hypothetical example with short sequences -** The following illustrates the disclosed methods using artificial, short sequences not intended to correspond to any particular real species. It is desired to fabricate a resequencing microarray for detection of species A, B, C, D, and E. As used herein, "species" may refer to taxonomic species as well as different types or strains of a single species, and combinations thereof. It is known that nominal target 1 (Fig. 5) is found in the genome of at least one of these species. A search for similar sequences is performed using a database such as BLAST to produce a list of targets. A minimum percent similarity, for example 70%, may be used to filter the results. If too many targets or targets from too many species, such as genetically distant species, are reported, the percentage may be raised to reduce the size of the list. Also, the list may be manually reviewed for removal of specific, undesirable targets.

Fig. 5 shows a hypothetical list of targets 10-40. (Reference number ranges such as "10-40" include only numbers of that form, rather than every number from 10 to 40.) The list of targets is provided to a computer system, which may be the same computer used to create the list. The list and all subsequently described data in this example at least up to assembling base call sequences is stored in a computer memory or medium. The list of candidate prototype sequences 100-400 in this example is identical to the list of targets 10-40, though that is not required.

Fig. 6 shows a hypothetical collection of probes 111-434 derived from the candidate prototype sequences 100-400. The subsequence length of the probes is chosen to be seven, though other values may be used. Probes 111-134 are derived from candidate prototype 100, and so forth. Probe 111 is the first seven bases of candidate prototype 100. Probes 112-114 are single nucleotide polymorphisms of probe 111 at the center position. Probes 111-114 make up one set of probes. Probes 121 and 131 are also seven base subsequences of candidate prototype 100, each shifting one base to the right. Thus, all three possible seven base subsequences of candidate prototype 100 are in the collection of probes. Probes 122-124 and 132-134 are single nucleotide polymorphisms of probes 121 and 131, respectively.

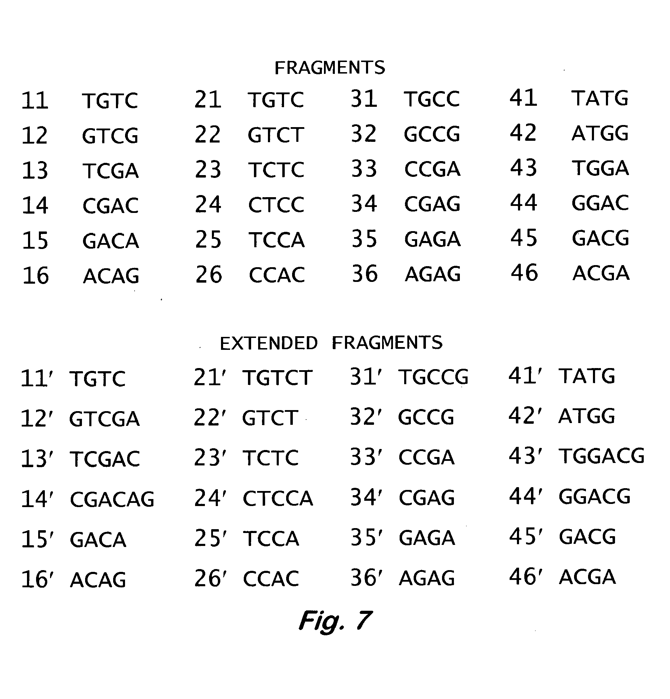

Fig. 7 shows a hypothetical list of fragments 11-46 derived from the targets 10-40. The fragment length is chosen to be 4, though other values may be used. Thus a target with a length of nine has six possible fragments. Also shown is a list of extended fragments 11'-46', containing some original fragments and some fragments made by adding extra bases from the target. The extended fragments are made by calculating the binding free energy of each fragment with a perfect complimentary sequence of the fragment. If the binding free energy for a fragment is above a predetermined, fixed threshold, the fragment is extended one nucleotide at a time until the binding free energy is below the threshold or the fragment is the same length as the probe. One suitable method of calculating the binding free energy is an oligonucleotide nearest neighbor model, though other methods may be used. A suitable binding free energy threshold for use with Affymetrix resequencing arrays is about -14.5 kcal/mol, though other values may be used. (No actual calculation was performed for this example, as it is illustrative.)

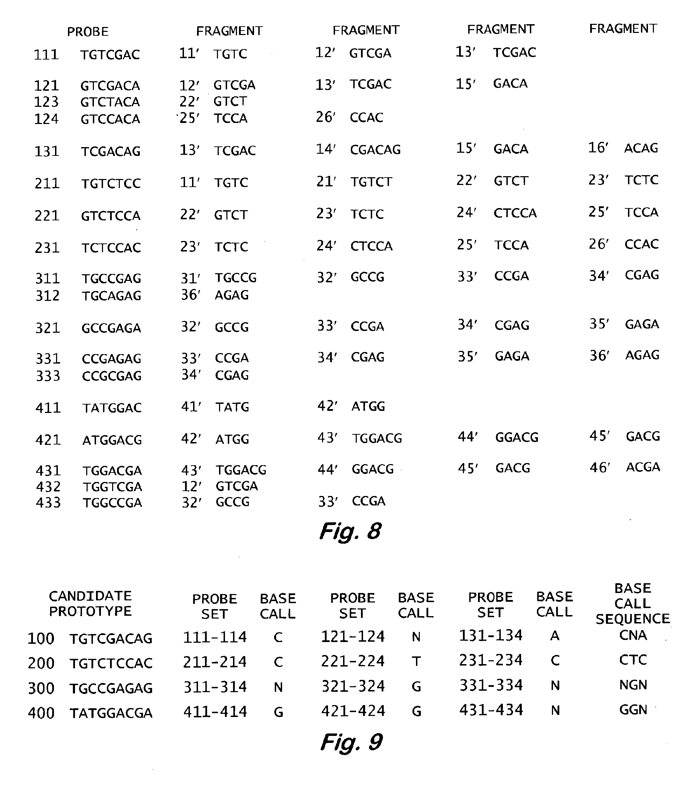

Fig. 8 shows all perfect matches between the probes and the extended fragments. The probe sets starting with 111, 131, 211, 221, 231, 321, 411, and 421 contain only one probe that matches any extended fragments. When assembling a base call sequence, these sets produce a base call that is the same as the center base of the first (non-polymorph) probe of the set. The probe sets starting with 121, 311, 331, and 431 contain more than one probe that matches any extended fragments. A non-base call ("N") is assigned for these probe sets. If there were any probe sets with no matches, these would also be assigned a non-base call. The base call sequence for each candidate prototype sequence and the probe sets from which they are derived are shown in Fig. 9.

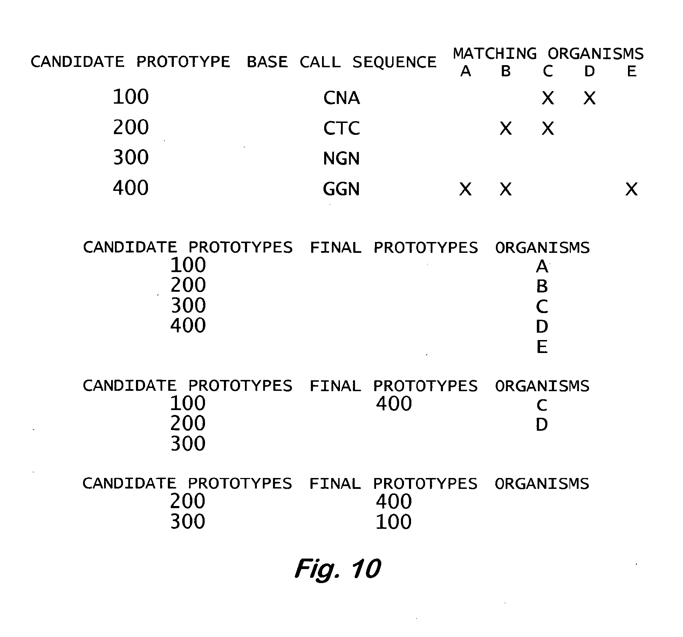

Fig. 10 shows the lists of matching organisms for each candidate prototype. The checked organisms contain the corresponding candidate prototype. This may be determined by reference to external databases. The minimum number of base calls is chosen to be 2, though larger numbers, such as 50, may be used. As such, no list of matching organisms need be made for candidate prototype 300, as its base call sequence (NGN) contains only one base call. This is the case even if it would match the most organisms. Candidate prototype 400 matches the most organisms (A, B, and E). It is added to the list of final prototypes and removed from the list of candidate prototypes. A, B, and E are removed from the list of organisms. At this point candidate prototype 100 matches two of the remaining organisms (C and D) while candidate prototype 200 matches only one (C). Candidate prototype 100 is added to the list of final prototypes and removed from the list of candidate prototypes. C and D are removed from the list of organisms. As the list of organisms is now empty, no more prototypes are moved to the list of final prototypes.

A resequencing microarray may be fabricated containing each set of probes corresponding to each final prototype sequence. Here the microarray would contain probes 111, 112, 113, 114, 121, 122, 123, 124, 131, 132, 133, 134, 411, 412, 413, 414, 421, 422, 423, 424, 431, 432, 433, and 434. This set of probes will detect each organism even though it does not detect every target. The microarray may also contain a sequence complimentary to each of these probes.

This example is based on a single nominal target, but more than one nominal target may be used. The target sequences may correspond to a single gene in common to a subset of the organisms, and the list of organisms may comprise a plurality of strains of a single species. If the resulting list of final prototypes cannot detect all the organisms, then the process or parts thereof may be repeated with different parameters, such as targets, candidate prototypes, probe length, fragment length, and minimum number of base calls.

### Example 2

**Enteroviruses and Adenoviruses -** The process was performed using enteroviruses and adenoviruses as the list of organisms. The final prototypes sequences are identified as SEQ ID NOS: 14-51. A resequencing microarray containing the probe sets generated from these prototypes was made has been designated RPMv.3.

Obviously, many modifications and variations of the present invention are possible in light of the above teachings. It is therefore to be understood that the claimed invention may be practiced otherwise than as specifically described. Any reference to claim elements in the singular, e.g., using the articles "a," "an," "the," or "said" is not construed as limiting the element to the singular.

## Claims

1. A computer-implemented method for selecting probes for resequencing microarrays comprising:
providing a list of target sequences associated with one or more organisms in a list of organisms;
providing a list of candidate prototype sequences suspected of hybridizing to one or more of the target sequences;
generating a collection of probes corresponding to each candidate prototype sequence, each collection of probes comprising a set of probes for every subsequence having a predetermined, fixed subsequence length of the corresponding candidate prototype sequence, the set consisting of the corresponding subsequence and every variation of the corresponding subsequence formed by varying a center nucleotide of the corresponding subsequence;
generating a set of fragments corresponding to each target sequence, each set of fragments comprising every fragment having a predetermined, fixed fragment length of the corresponding target sequence;
calculating the binding free energy of each fragment with a perfect complimentary sequence of the fragment, and if any binding free energy is above a predetermined, fixed threshold, the fragment is extended one nucleotide at a time until the binding free energy is below the threshold or the fragment is the same length as the probe, generating a set of extended fragments; and
determining which extended fragments are perfect matches to any of the probes; and
assembling a base call sequence corresponding to each candidate prototype sequence comprising:
a base call corresponding to the center nucleotide of each probe of the corresponding prototype sequence that is a perfect match to any extended fragment, but for which the other members of the set of probes containing the perfect match probe are not perfect matches to any extended fragment; and
a non-base call in all other circumstances.

2. The method of claim 1, wherein a subset of the target sequences corresponds to a single gene in common to a subset of the organisms.

3. The method of claim 1, wherein the list of organisms comprises a plurality of strains of a single species.

4. The method of claim 1, further comprising:
selecting a nominal target sequence known to be found in at least one of the organisms;
performing a similarity search against a database of known sequences to add additional target sequences having at least a predetermined degree of similarity to the nominal target sequence; and
generating the list of candidate prototype sequences to be identical to the list of target sequences.

5. The method of claim 1, wherein the subsequence length is 25.

6. The method of claim 1, wherein the fragment length is 13.

7. The method of claim 1, wherein the binding free energy is calculating according to an oligonucleotide nearest neighbor model.

8. The method of claim 1, wherein the binding free energy threshold is about -14.5 kcal/mol.

9. The method of claim 1, further comprising:
generating for each candidate prototype sequence a list of matching organisms containing that candidate prototype sequence for which the base call sequence corresponding to that candidate prototype sequence comprises a fixed minimum number of base calls;
moving the candidate prototype sequence corresponding to the longest list of matching
organisms to a list of final prototype sequences;
removing the matching organisms corresponding to the moved prototype sequence from
the list of organisms; and
repeating the moving and removing until the list of organisms is empty.

10. The method of claim 9, wherein the minimum number of base calls is 50.

11. The method of claim 9, further comprising:
fabricating a resequencing microarray containing each set of probes corresponding to each final prototype sequence.

12. The method of claim 11, wherein the microarray further comprises a sequence complimentary to each probe on the microarray.

## Patentansprüche

1. Computer-implementiertes Verfahren zum Auswählen von Sonden zur Re-Sequenzierung von Mikroarrays, welches Folgendes umfasst:
Bereitstellen einer Liste von Zielsequenzen im Zusammenhang mit einem oder mehreren Organismen in einer Liste von Organismen;
Bereitstellen einer Liste von Kandidatenprototypsequenzen, von welchen vermutet wird, dass sie zu einer oder mehreren der Zielsequenzen hybridisieren;
Erzeugen einer Sammlung von Sonden, welche jeder Kandidatenprototypsequenz entsprechen, wobei jede Sammlung von Sonden einen Satz Sonden für jede Untersequenz mit einer vorbestimmten, feststehenden Untersequenzlänge der entsprechenden Kandidatenprototypsequenz umfasst, wobei der Satz aus der entsprechenden Untersequenz und jeder Variation der entsprechenden Untersequenz gebildet durch Variieren eines Mittelnukleotides der entsprechenden Untersequenz besteht;
Erzeugen eines Satzes von Fragmenten, welcher jeder Zielsequenz entspricht, wobei jeder Satz von Fragmenten jedes Fragment mit einer vorbestimmten, feststehenden Fragmentlänge der entsprechenden Zielsequenz umfasst;
Berechnen der bindungsfreien Energie von jedem Fragment mit einer perfekten komplementären Sequenz des Fragmentes und, wenn eine bindungsfreie Energie über einer vorbestimmten, feststehenden Schwelle liegt, Erweitern des Fragmentes ein Nukleotid nach dem anderen, bis die bindungsfreie Energie unter der Schwelle liegt oder das Fragment die gleiche Länge wie die Sonde aufweist, wodurch ein Satz erweiterter Fragmente erzeugt wird; und
Bestimmen, welche erweiterten Fragmente perfekte Übereinstimmungen mit einer der Sonden sind; und
Zusammenstellen einer Base-Call-Sequenz, welche jeder Kandidatenprototypsequenz entspricht, welche Folgendes umfasst:
einen Base-Call, welcher dem Mittelnukleotid jeder Sonde der entsprechenden Prototypensequenz entspricht,
bei welcher es sich um eine perfekte Übereinstimmung mit einem erweiterten Fragment handelt, aber für welche die anderen Mitglieder des Satzes von Sonden,
der die perfekt übereinstimmende Sonde enthält, keine perfekten Übereinstimmungen mit einem erweiterten Fragment sind; und
einen Nicht-Base-Call in allen anderen Fällen.

2. Verfahren nach Anspruch 1, wobei ein Untersatz der Zielsequenzen einem einzelnen Gen entspricht, das einem Untersatz der Organismen gleich ist.

3. Verfahren nach Anspruch 1, wobei die Liste der Organismen mehrere Stämme einer einzelnen Spezies umfasst.

4. Verfahren nach Anspruch 1, welches ferner Folgendes umfasst:
Auswählen einer nominalen Zielsequenz, von welcher bekannt ist, dass sie in mindestens einem der Organismen vorkommt;
Durchführen einer Ähnlichkeitssuche in einer Datenbank bekannter Sequenzen zum Hinzufügen zusätzlicher Zielsequenzen mit mindestens einem vorbestimmten Ähnlichkeitsgrad zu der nominalen Zielsequenz; und
Erzeugen der Liste von Kandidatenprototypsequenzen, die identisch mit der Liste der Zielsequenzen sein soll.

5. Verfahren nach Anspruch 1, wobei die Untersequenzlänge 26 ist.

6. Verfahren nach Anspruch 1, wobei die Fragmentlänge 13 ist.

7. Verfahren nach Anspruch 1, wobei die bindungsfreie Energie gemäß einem Nächster-Nachbar-Oligonukleotid-Modell berechnet wird.

8. Verfahren nach Anspruch 1, wobei die Schwelle der bindungsfreien Energie etwa -14,5 kcal/Mol beträgt.

9. Verfahren nach Anspruch 1, welches ferner Folgendes umfasst:
für jede Kandidatenprototypsequenz, Erzeugen einer Liste übereinstimmender Organismen, welche diese Kandidatenprototypsequenz enthalten, für welche die Base-Call-Sequenz, welche dieser Kandidatenprototypsequenz entspricht, eine feststehende Mindestanzahl an Base-Calls umfasst;
Bewegen der Kandidatenprototypsequenz, welche der längsten Liste übereinstimmender Organismen entspricht, zu einer Liste abschließender Prototypsequenzen;
Entfernen der übereinstimmenden Organismen, welche der bewegten Prototypsequenz entsprechen, aus der Liste von Organismen; und
Wiederholen des Bewegens und Entfernens, bis die Liste der Organismen leer ist.

10. Verfahren nach Anspruch 9, wobei die Mindestanzahl an Base-Calls 50 ist.

11. Verfahren nach Anspruch 9, welches ferner Folgendes umfasst:
Herstellen eines Re-Sequenzierungs-Mikroarrays, welches jeden Satz Sonden enthält, der jeder abschließenden Prototypsequenz entspricht.

12. Verfahren nach Anspruch 11, wobei das Mikroarray ferner eine Sequenz umfasst, welche komplementär zu jeder Sonde auf dem Mikroarray ist.

## Revendications

1. Procédé implémenté par ordinateur de sélection de sondes pour micropuces de reséquençage, ledit procédé comprenant :
la fourniture d'une liste de séquences cibles associées à un ou plusieurs organismes figurant dans une liste d'organismes ;
la fourniture d'une liste de séquences prototypes candidates suspectées d'hybridation à une ou plusieurs des séquences cibles ;
la génération d'un recueil de sondes correspondant à chaque séquence prototype candidate, chaque recueil de sondes comprenant un ensemble de sondes pour chaque sous-séquence d'une longueur de sous-séquence fixe prédéterminée de la séquence prototype candidate correspondante, l'ensemble étant constitué de la sous-séquence correspondante et de chaque variation de la sous-séquence correspondante formée en modifiant un nucléotide central de la sous-séquence correspondante ;
la génération d'un ensemble de fragments correspondant à chaque séquence cible, chaque ensemble de fragments comprenant tous les fragments d'une longueur de fragment fixe et prédéterminée de la séquence cible correspondante ;
le calcul de l'énergie libre de liaison de chaque fragment avec une séquence parfaitement complémentaire du fragment, et si une quelconque énergie libre de liaison est au-dessus d'un seuil fixe prédéterminé, le fragment est étendu d'un nucléotide à la fois jusqu'à ce que l'énergie libre de liaison soit en-deçà du seuil ou jusqu'à ce que le fragment soit de la même longueur que la sonde, générant un ensemble de fragments étendus ; et
la détermination des fragments étendus qui sont des correspondances parfaites avec l'une quelconque des sondes ; et
l'assemblage d'une séquence d'appel de base correspondant à chaque séquence prototype candidate,
comprenant :
un appel de base correspondant au nucléotide central de chaque sonde de la séquence prototype correspondante qui correspond parfaitement à un fragment étendu quelconque, mais pour lequel les autres éléments de l'ensemble de sondes contenant la sonde de correspondance parfaite ne sont des correspondances parfaites avec aucun fragment étendu ; et
un appel non base dans toutes les autres circonstances.

2. Procédé selon la revendication 1, dans lequel un sous-ensemble des séquences cibles correspond à un gène unique en commun avec un sous-ensemble des organismes.

3. Procédé selon la revendication 1, dans lequel la liste d'organismes comprend une pluralité de souches d'une seule espèce.

4. Procédé selon la revendication 1, comprenant en outre :
la sélection d'une séquence cible nominale connue pour être rencontrée dans au moins l'un des organismes ;
la réalisation d'une recherche de similarité dans une base de données de séquences connues pour ajouter des séquences cibles supplémentaires ayant au moins un degré prédéterminé de similarité avec la séquence cible nominale ; et
la génération de la liste de séquences prototypes candidates pour qu'elle soit identique à la liste de séquences cibles.

5. Procédé selon la revendication 1, dans lequel la longueur de la sous-séquence est de 25.

6. Procédé selon la revendication 1, dans lequel la longueur du fragment est de 13.

7. Procédé selon la revendication 1, dans lequel l'énergie libre de liaison est calculée en fonction d'un modèle du plus proche voisin oligonucléotidique.

8. Procédé selon la revendication 1, dans lequel le seuil d'énergie libre de liaison est d'environ - 14,5 kcal/mol.

9. Procédé selon la revendication 1, comprenant en outre :
la génération pour chaque séquence prototype candidate d'une liste d'organismes correspondants contenant la séquence prototype candidate pour laquelle la séquence appel de base correspondant à la séquence prototype candidate comprend un nombre minimal fixe d'appels de base ;
le déplacement dans une liste de séquences prototypes finales de la séquence prototype candidate correspondant à la plus longue liste d'organismes correspondants ;
le retrait des organismes correspondants qui correspondent à la séquence prototype déplacée de la liste d'organismes ; et
la répétition du déplacement et du retrait jusqu'à ce que la liste d'organismes soit vide.

10. Procédé selon la revendication 9, dans lequel le nombre minimal d'appels de base est de 50.

11. Procédé selon la revendication 9, comprenant en outre :
la fabrication d'une micropuce de reséquençage contenant chaque ensemble de sondes correspondant à chaque séquence prototype finale.

12. Procédé selon la revendication 11, dans lequel la micropuce comprend en outre une séquence complémentaire de chaque sonde sur la micropuce.
